# NEUE EUROPÄISCHE PATENTSCHRIFT

(11) **EP 2 570 109 B2**
(45) Veröffentlichungstag und Bekanntmachung des Hinweises auf die Entscheidung über den Einspruch: **22.12.2021**
(45) Hinweis auf die Patenterteilung: 25.07.2018
(21) Anmeldenummer: 11188014.2
(22) Anmeldetag: 07.11.2011
(51) Int. Cl.: A61K 8/04, A61K 8/31, A61K 8/49, A61Q 17/04

(54) **Kosmetisches Sonnenschutzspray**
Cosmetic sun protection spray
Spray de protection solaire cosmétique

(30) Priorität: 10.11.2010 DE 102010050774
(43) Veröffentlichungstag der Anmeldung: 20.03.2013
(73) Patentinhaber: Beiersdorf AG, 20253 Hamburg (DE)
(72) Erfinder: Zanforlin Trede, Lucia, 22769 Hamburg (DE); Gronau, Annette, 22549 Hamburg (DE)
(74) Vertreter: Beiersdorf AG

(56) Entgegenhaltungen:
- EP-A1- 1 508 326
- EP-A1- 2 027 847
- EP-B1- 1 549 281
- EP-B1- 2 276 451
- WO-A1-2004/000243
- WO-A1-2006/034996
- WO-A1-2006/099687
- WO-A1-2007/002047
- WO-A1-2008/080892
- WO-A2-03/051522
- DE-A1- 10 247 695
- DE-A1-102004 003 436
- DE-A1-102004 047 287
- US-A1- 2009 041 686
- US-B1- 6 423 302
- US-B1- 7 252 816
- DATABASE GNPD [Online] MINTEL; 1. September 2006 (2006-09-01), Beiersdorf Nivea Sun: "Pampering Protection Mousse SPF 15", XP002756648, Database accession no. 581323
- "Atomizing sprays for sun care applications", IP.COM JOURNAL, IP.COM INC., WEST HENRIETTA, NY, US, 30 November 2006 (2006-11-30), XP013116940, ISSN: 1533-0001
- GNPD-Datenbank, Record ID 942451

## Beschreibung

Die vorliegende Erfindung betrifft eine kosmetische Zubereitung enthaltend 2,4-Bis-{[4-(2-ethyl-hexyloxy)-2-hydroxy]-phenyl}-6-(4-methoxyphenyl)-1,3,5-triazin sowie Propan und/oder Butan.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.
Ein vielfach in kosmetischen Zubereitungen eingesetzter UV-Filter ist die Verbindung 2,4-Bis-{[4-(2-ethyl-hexyloxy)-2-hydroxy]-phenyl}-6-(4-methoxyphenyl)-1,3,5-triazin (INCI :Bis-Ethylhexyloxyphenol Methoxyphenyl Triazin), welche beispielsweise unter der Bezeichnung Tinosorb S bei der Firma BASF (ehemals Ciba) erhältlich ist. Dieser UV-Filter, der in der Regel als Breitbandfilter eingesetzt wird, hat jedoch u.a. den Nachteil, eine starke gelbliche Eigenfärbung aufzuweisen. Darüber hinaus ist er, bekanntermaßen, relativ schwer löslich.

Die schwere Löslichkeit und die markante Eigenfarbe haben nun den Nachteil, dass mit Sonnenschutzmittel, die diesen Filter enthalten, sich relativ schwer aus mit dem Sonnenschutzmittel kontaminierten Kleidungsstücken (T-Shirts, Badelaken, Handtücher) wieder herauswaschen lassen. Wenn nun von den Konsumenten beispielsweise im Sommer helle (z.B. weiße) Kleidung getragen oder entsprechend helle Handtücher am Strand oder Schwimmbad verwendet werden und der Anwender ein Bis-Ethylhexyloxyphenol Methoxyphenyl Triazin-haltiges Sonnenschutzmittel verwendet hat (welches in der Regel einen hohen Schutz vor der UV-Strahlung bewirkt), führt dies fast zwangsläufig zur Kontamination der Kleidung/des Handtuches mit dem Sonnenschutzmittel, wodurch das Textil eine, je nach Filtergehalt unterschiedlich starke Gelbfärbung erhält. Diese Gelbfärbung lässt sich nach dem Stand der Technik nur relativ schwer mit Waschmitteln wieder herauswaschen.
Es war daher die Aufgabe der vorliegenden Erfindung, ein Bis-Ethylhexyloxyphenol Methoxyphenyl Triazin haltiges Sonnenschutzmittel zu entwickeln, bei dem sich die UV-Filter, insbesondere Bis-Ethylhexyloxyphenol Methoxyphenyl Triazin leichter wieder herauswaschen lassen, bzw. eine geringere Gelbfärbung im Textil nach dem Waschvorgang zurücklassen.
Gelöst wird die Aufgabe durch eine kosmetische Emulsion gemäß Anspruch 1.

Zwar kennt der Stand der Technik beispielsweise die WO 02/03927 und WO 2007/002047, welche Sprayzubereitungen mit UV-Filtern und Propan/Butan offenbaren, doch konnten diese Schriften nicht den Weg zur Erfindung weisen, da die Zubereitungen nicht den erfindungsgemäßen, gelbstichigen UV-Filter enthalten.

Darüber hinaus kennt der Fachmann die EP 2031963, EP 1400571 und WO 2007/079807, sowie die DE 102008021631 und DE 102005059738, die ebenfalls nicht den Weg zur vorliegenden Erfindung weisen konnten.

Ferner kennt der Fachmann ip.com Internet-Veröffentlichung mit der Registrierungsnummer IPCOM000143596D vom 30.11.2006 mit dem Titel "Atomizing sprays for sun care applications", die nicht den Weg zur vorliegenden Erfindung weisen konnte.

Nicht zuletzt kennt der Fachmann die WO 2004/000243, US 7252816, US 2009/041686, DE 102004047287, EP 2027847 sowie den Datenbank-Eintrag in GNPD, Mintel Eintrag vom 1.9.2006 (No 581323) "Beiersdorf Nivea sun pampering protection mousse SPF 15" (XP002756648), die ebenfalls nicht den Weg zur vorliegenden Erfindung weisen konnten.

Es ist erfindungsgemäß, wenn die erfindungsgemäße Zubereitung in Form eines Aerosolsprays vorliegt. Diese werden erfindungsgemäß vorteilhaft in Weißblechdosen mit Sprühkopf aufbewahrt.

Erfindungsgemäß können als Butan sowohl n-Butan als auch Isobutan und deren Mischungen eingesetzt werden. Die Wahl des Butans bzw. -gemisches ist für die Ausführung der Erfindung weitgehend unkritisch. Es ist jedoch erfindungsgemäß, wenn die Zubereitung eine Mischung aus Propan/Butan enthält.

Erfindungsgemäß vorteilhafte Ausführungsformen der vorliegenden Erfindung sind dadurch gekennzeichnet, dass die Zubereitung 2,4-Bis-{[4-(2-ethyl-hexyloxy)-2-hydroxy]-phenyl}-6-(4-methoxyphenyl)-1,3,5-triazin in einer Menge von 0,5 bis 5 Gewichts-%, bezogen auf das Gesamtgewicht der Zubereitung, inklusive Propan/Butan, enthält. Dabei ist es erfindungsgemäß bevorzugt, wenn die Zubereitung 2,4-Bis-{[4-(2-ethyl-hexyloxy)-2-hydroxy]-phenyl}-6-(4-methoxyphenyl)-1,3,5-triazin in einer Menge von 1 bis 3 Gewichts-%, bezogen auf das Gesamtgewicht der Zubereitung, inklusive Propan/Butan, enthält.

Es ist erfindungsgemäß vorteilhaft, wenn die Zubereitung Propan/Butan (d.h. Propan und/oder Butan) in einer Gesamtmenge von 15 bis 45 Gewichts-%, bezogen auf das Gesamtgewicht der Zubereitung, enthält. Es ist erfindungsgemäß bevorzugt, wenn die Zubereitung Propan/Butan in einer Gesamtmenge von 20 bis 30 Gewichts-%, bezogen auf das Gesamtgewicht der Zubereitung, enthält.
Erfindungsgemäß vorteilhafte Ausführungsformen der vorliegenden Erfindung sind dadurch gekennzeichnet, dass das Gewichtsverhältnis von 2,4-Bis-{[4-(2-ethyl-hexyloxy)-2-hydroxy]-phenyl}-6-(4-methoxyphenyl)-1,3,5-triazin zur Gesamtmenge an Propan/Butan von 0,01 bis 0,2 beträgt.

Darüber hinaus ist vorteilhaft im Sinne der vorliegenden Erfindung, wenn die Zubereitung 4-(tert.-Butyl)-4'-methoxydibenzoylmethan und/oder 2-(4'-Diethylamino-2'-hydoxybenzoyl)-benzoesäurehexylester als weitere UV-Filter enthält. Dabei ist der alleinige Einsatz von 4-(tert.-Butyl)-4'-methoxydibenzoylmethan erfindungsgemäß bevorzugt.

4-(tert.-Butyl)-4'-methoxydibenzoylmethan und/oder 2-(4'-Diethylamino-2'-hydoxybenzoyl)-benzoesäurehexylester werden erfindungsgemäß vorteilhaft in einer Gesamtkonzentration von 2,5 bis 10 Gew.-%, bezogen auf das Gesamtgewicht der Zubereitung (inklusive Propan/Butan), eingesetzt.

4-(tert.-Butyl)-4'-methoxydibenzoylmethan und/oder 2-(4'-Diethylamino-2'-hydoxybenzoyl)-benzoesäurehexylester werden erfindungsgemäß bevorzugt in einer Gesamtkonzentration von 4 bis 8 Gew.-%, bezogen auf das Gesamtgewicht der Zubereitung (inklusive Propan/Butan), eingesetzt. Enthält die Zubereitung nur einen der beiden Filter, bezieht sich die Konzentrationsangabe allein auf diesen einen Filter.

Erfindungsgemäß vorteilhafte Ausführungsformen enthalten dabei von 5 bis 10 Gew.-% 2-Ethylhexyl-2-cyano-3,3-diphenylacrylat (Octocrylen), bezogen auf das Gesamtgewicht der Zubereitung (inklusive Propan/Butan), erfindungsgemäß bevorzugte Ausführungsformen enthalten von 7 bis 9 Gew.-% 2-Ethylhexyl-2-cyano-3,3-diphenylacrylat (Octocrylen), bezogen auf das Gesamtgewicht der Zubereitung (inklusive Propan/Butan).

Darüber hinaus ist es erfindungsgemäß vorteilhaft, wenn die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze;2-Methyl-5-(2-oxo-3-bornylidenmethyl)sulfonsäuresalze;2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimt-säure(2-ethylhexyl)ester; 4-Methoxyzimtsäureisoamylester; 2-Hydroxy-4-methoxybenzophenon, 2-Hydroxy-4-meth-oxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxy-siloxan / Dimethylsiloxan - Copolymer; Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Titandioxid; Zinkoxid.

Es ist jedoch erfindungsgemäß bevorzugt, wenn die Zubereitung frei ist von Titandioxid, Zinkoxid, 3-(4-Methylbenzyliden)campher und Polysilicon-15.

Die erfindungsgemäß bevorzugte Ausführungsform der Emulsion ist die Öl-in-Wasser-Emulsion (O/W-Emulsion).

Liegt die erfindungsgemäße Zubereitung in Form einer O/W-Emulsion vor, so ist es erfindungsgemäß bevorzugt, wenn die Zubereitung in Form einer O/W-Emulsion vorliegt, die einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat , Natriumcetearylsulfat enthält.

Erfindungsgemäß bevorzugt sind dabei Zubereitungen, die keine PEG (Polyethylenglycole) oder PEG-Derivate enthalten.

Es ist erfindungsgemäß vorteihaft, wenn die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, Coffein, Menthol, β-Alanin, Tocopherylacetat, Harnstoff; Hyaluronsäure; Dihydroxyaceton; 8-Hexadecen-1,16-dicarbonsäure, Glycyrrhetinsäure, Glucosylglyceride und/oder Licochalcon A enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9), Talkum, Lauroyl Lysine und Acrylonitrile-methacrylonitrile-methyl-methacrylate.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl wie Isopropanol, Diole oder Polyole niedriger C-Zahl sowie deren Ether, vorzugsweise Propylenglykol, 2-Methylpropan-1,3-diol, Pentan-1,2-diol, Hexan-1,2-diol, Octan-1,2-diol, Decan-1,2-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Schaumstabilisatoren, Elektrolyte, etc.. Die erfindungsgemäße Zubereitung kann vorteilhaft Konsistenzbildner (Gelbildner, Verdickungsmittel) wie beispielsweise Polyacrylate (auch quervernetzt) oder Cellulosederivate (beispielsweise Hydroxyethylcellulose) oder andere enthalten.

Selbstverständlich kann die Zubereitung mit den üblichen, in der Kosmetik verwendeten Konservierungsmitteln konserviert werden.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyl-lactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*)*.*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten, wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Die erfindungsgemäßen Zubereitungen werden erfindungsgemäß bevorzugt als Sonnenschutzmittel verwendet. Darüber hinaus ist ihr Einsatz als Tagespflegeprodukt erfindungsgemäß.

### Vergleichsversuche

Mit dem folgenden Vergleichsversuch konnte der erfindungsgemäße Effekt belegt werden:
Sonnenschutzprodukte führen zu teilweise starken gelblichen Verfleckungen der Textilien. Dies ist auf die UV-Filter rückzuführen.

Die rein visuelle Abmusterung von Textilien kann durch die Durchführung einer technischen Farbmessung ergänzt werden. Vorteil der maschinellen Farbmessung ist die schnelle Durchführbarkeit und die Unabhängigkeit von Personen sowie die leichtere Quantifizierung und Dokumentierbarkeit.

Die genaue zahlenmäßige Beschreibung von Farbzuständen erfordert die Definition von Farbsystemen. Heute üblich ist die Verwendung des CIE-Lab-Systems.

### Eingesetzte Methode

1. Mit einer Zackenschere werden ca. 15x20 cm große Lappen aus weißer Jersey-Baumwolle zugeschnitten.
2. Textilien vorwaschen mit 1 g Waschmittel und 300 mL Leitungswasser: 1x 1,5 h (1h bei 60 °C, dann 30 min bei 30 °C), Gerät: Atlas, Linitest.
3. Textilien unter fließendem Leitungswasser auswaschen, schleudern und trocknen
4. 1 g der Analysensubstanz (Sonnenschutzmittel) auf eine 5x5 cm große PMMA Platte auftragen und gleichmäßig auf den Stoff aufdrucken, an der Luft trocknen lassen
5. Messung der Textilflecken mit dem Farbmessgerät des Modells "Spectro-Color"; Dr. Lange in der Auflösung d/8°
6. Nach der Beurteilung der Stofflappen erneut waschen, nach dem Waschen unter fließendem Wasser ausspülen, schleudern und trocknen
7. Erneute Messung der Flecken mit dem Farbmessgerät

### Formulierung (Wirklösung)

| | **Rezeptur** |
|---|---|
| **INCI-Name(n)** | **m [%]** |
| Octocrylene | 7.0000 |
| **Bis-Ethylhexyloxyphenol Methoxyphenyl Triazine** | **2.5000** |
| Butyl Methoxydibenzoylmethane | 4.5000 |
| Phenylbenzimidazole Sulfonic Acid | 1.5000 |
| Weitere UV-Filter wie z.B. Phenylbenzimidazole Sulfonic Acid, Ethylhexyl Methoxycinnamate, Homosalate und Ethylhexyl Salicvlate | 5.0000 |
| Komplexbildner | 1.0000 |
| Verdicker | 0.4000 |
| Moisturizer | 10.000 |
| Neutralisationsmittel | 0.3300 |
| Filmbildner | 0.5000 |
| Emulgator | 1.0000 |
| Öle | 9.0000 |
| Wachse | 1.0000 |
| Alkohole | 8.0000 |
| Konservierungsmittel | 1.0000 |
| Parfüm | 0.4000 |
| Aqua | Ad 100,0 |

### Durchführung

Die beschriebene Methode wird mit der Rezeptur 1 angewendet. Diese Formulierung wird zum Einen mit einer Pipette aufgetragen (MUSTER 1) und zum Anderen aus einer Aerosoldose auf eine PMMA-Platte gesprüht (MUSTER 2). Das Aerosolprodukt besteht aus 25 Gew.-% Propan/Butan 2.7 als Treibgas und 75 Gew.-% Wirklösung (Formulierung).

Um die Menge von 1 g Wirklösung auf der gesprühten Platte zu gewährleisten, wird in Abständen von 15 Minuten aus der Aerosoldose nachgesprüht.

### Auswertung und Ergebnisse

Für die Beurteilung einer Verfleckung ist die Differenz der Gesamtfarbtöne von Fleckareal und Umgebung von Interesse. Aus Gründen der besseren Handhabbarkeit werden die Differenzen der einzelnen Koordinaten zum **Gesamtfarbabstand ΔE*** zusammengefasst, wodurch allerdings die Aussagen über die einzelnen Anteile des Farbeindrucks verloren gehen. Aus diesem Grund wird der **Maßzahl b*** für die Beurteilung der gelblichen Verfleckung herangezogen. Die Maßzahlen beschreiben den Farbton. Eine positive Maßzahl b* kennzeichnet gelb. Je höher die Zahl ist desto gelber der Eindruck des Betrachters.

| | **Vor dem Waschen** | | **Nach dem Waschen** | | **Differenz** | |
|---|---|---|---|---|---|---|
| | **Gesamtfarbeindruck (E*)** | **Maßzahl (b*)** | **Gesamtfarbeindruck (E*)** | **Maßzahl (b*)** | **Gesamtf arbabstand (ΔE*)** | **Maßzahl (Δb*)** |
| Muster 1 | 13,33 | 12,76 | 10,24 | 8,46 | -23,18 | -33,69 |
| Muster 2 | 12,73 | 12,17 | 9,56 | 7,83 | -24,90 | -35,66 |

Beide Muster haben eine positive Maßzahl b* (= gelb). Jedoch weist Muster 2, das Aerosolprodukt, sowohl vor als auch nach dem Waschen einen kleineren Wert als Muster 1 auf. Zudem ist die Differenz von vor zu nach dem Waschen bei Muster 2 größer, d.h. dass die geringere gelbliche Textilverfärbung des Musters 2 sich sogar besser auswaschen lässt als diejenige des ersten Musters.

Wird der Gesamtfarbabstand ΔE* beider Produkte verglichen, ist festzustellen, dass Muster 2 einen geringeren Farbeindruck aufweist. Die Differenz vor und nach dem Waschen bei Muster 1 ist geringer als bei Muster 2 und somit lässt sich festhalten, dass die verursachte Verfleckung auch nach dem Waschen von Muster 2 geringer ist.

### Fazit

Die dünnflüssige o. g. Formulierung wurde in einer Aerosoldose mit Propan/Butan als Treibgas abgefüllt. Die gleiche Formulierung wurde so in 2 unterschiedlichen Anwendungsformen nach der Textilverfleckung untersucht. Überraschenderweise wurde festgestellt, dass das Aerosolprodukt eine geringere Textilverfärbung verursacht. Es ist davon auszugehen, dass Propan/Butan einen entscheidenden Einfluss auf die Verfleckung von Textilien hat und so dafür sorgt, dass eine geringere Verfleckung auftritt.

### Beispiele

Die nachfolgenden Beispiele sollen die vorliegende Erfindung verdeutlichen ohne sie einzuschränken. Die Angaben beziehen sich stets auf Gewichts-%, sofern nicht andere Angaben gemacht werden.

Aus den angegebenen Rezepturen werden Aerosolprodukte hergestellt. Diese Aerosolprodukte bestehen aus 25 Gew.-% Propan/Butan 2.7 als Treibgas und 75 Gew.-% Wirklösung (Formulierung von 1 bis 10). Die Aerosolprodukte werden in handelsübliche Aerosoldosen abgefüllt.

| | **1** | **2** | **3** | **4** | **5** | **6** | **7** | **8** | **9** | **10** |
|---|---|---|---|---|---|---|---|---|---|---|
| Acrylat/C10-30 Alkyl Acrylat Crosspolymer | | 0,3 | | 0,3 | 0,1 | | 0,25 | 0,2 | 0,2 | 0,2 |
| Carbomer | 0,2 | | 0,2 | | | | | 0,15 | | |
| Xanthan Gummi | 0,2 | 0,3 | | | | | | | | 0,2 |
| Copolymer aus Vinylpyrrolidon und Acrylsäure | | | 0,4 | | | 0,3 | | | | |
| Tapioca Stärke | 1 | | | | | | | | | |
| Distarch Phosphate | | | 1 | | | | | | | |
| Carrageenan | | | | | 0,2 | | | | 0,25 | |
| VP/Hexadecene Copolymer | 1,5 | 1 | | 0,5 | 0,5 | 0,5 | 1 | 0,75 | 1 | 0,5 |
| C18-36 Acid Triglyceride | | | 1 | | | 1 | | 0,5 | | 1 |
| Natrium Stearoylglutamat | 0,2 | | | | | | | | | 0,1 |
| Sucrosepolystearat | 1 | | | | | | | | | 1 |
| Glyceryl Stearat | | | | | | | | 1 | | |
| Sodium Cetearyl Sulfate | | | | | | 1,5 | | | | |
| Glyceryl Stearat SE | | | | | | 0,5 | | | | |
| Glyceryl Stearate Citrate | | 1 | | | | | | | | |
| Ceteareth-20 | | | 1 | 1 | 1 | | 1 | | 1 | |
| Dibutyl Adipate | | | 4 | | | 2 | | | | 2 |
| Myristyl Myristat | | | | | | 0,5 | | | | |
| Butylenglycol Dicaprylat/ Dicaprat | 5 | | 4 | | | 3 | | 3,5 | 6 | |
| C12-15 Alkyl Benzoat | 1,5 | | | 2 | | 2 | 3 | | | |
| Caprylic/Capric Triglycerid | | | | 2 | | | | | | 3 |
| Cetearylalkohol | | | | | | | | | | 0,2 |
| Cyclomethicon | 0,5 | | 0,5 | | | | | | 0,5 | 0,5 |
| Dicaprylylcarbonat | | 3 | | | 3 | | | | | |
| Octyldodekanol | 3 | | | | 3,5 | | 4 | 3,5 | | 2 |
| Coco-Caprylate | | 5,5 | | | 2 | 3 | | | | 1 |
| Isopropyl Palmitate | | | | 3 | | | 2 | | 1 | 2 |
| Bis- Ethyl hexyloxyphenol Methoxyphenyl Triazin | 2 | 1 | 1,5 | 2,5 | 0,5 | 3 | 1,5 | 1 | 2 | 2 |
| Butyl Methoxydibenzoylmethan | 4,5 | 3 | | 4 | 4,5 | | 4 | 3 | 4 | 2 |
| Diethylamino Hydroxybenzoyl Hexyl Benzoat | | 2 | 8 | | | 6 | | 1 | | 6 |
| Ethylhexyl Methoxycinnamat | 5 | | | | | | | | 0,5 | |
| Ethylhexyltriazone | | | | | 0,5 | 1 | | | | |
| Homosalat | | 4 | | 5 | | | 2 | | | 2 |
| Octocrylen | 5 | 6 | 8 | 6,5 | 7,5 | 6 | 9 | 7 | 6 | 10 |
| Octylsalicylat | | | | 3 | | 4 | 2 | 1 | | |
| Phenylbenzimidazol Sulfonsäure | 1 | | 1,5 | | | | 2 | | 1,5 | |
| Tris-(biphenyl)-1,3,5 triazin | | | | 2 | | | | | 1 | |
| Tocopherol Acetate | 0,5 | 1 | 0,5 | 0,5 | 1 | 0,5 | 1 | 0,5 | 1 | 0,5 |
| Glycerin | 5 | 6 | 10 | 9 | 8 | 7 | 7 | 9 | 10 | 12 |
| Glycyrrhetinsäure | 0,1 | | | | | | | | 0,2 | |
| Ubiquinone | | 0,3 | | | | | | | | |
| Alcohol Denat. | 6 | 8 | 7 | 9 | 10 | 7 | 8 | 8 | 6 | 7 |
| Methylparaben | | | | 0,1 | | 0,2 | 0,3 | | | |
| Ethylparaben | 0,2 | | 0,2 | | 0,1 | | | | 0,3 | |
| Ethylhexyl Glycerin | | 0,5 | 1 | | 0,5 | | | 1 | | |
| 2-Methylpropan-1-3-diol | 0,2 | | | | | | | | | 0,4 |
| EDTA | 1 | 1 | 1 | 1 | 1 | 1 | 1 | 1 | 1 | 1 |
| Neutralisationsmittel (z.B. NaOH) | q.s. | q.s. | q.s. | q.s. | q.s. | q.s | q.s | q.s | q.s | q.s |
| Wasser | ad 100 | ad 100 | ad 100 | ad 100 | ad 100 | ad 100 | ad 100 | ad 100 | ad 100 | ad 100 |

## Patentansprüche

1. Kosmetische Emulsion die in Form eines Aerosolsprays vorliegt, enthaltend
a) 2,4-Bis-{[4-(2-ethyl-hexyloxy)-2-hydroxyl-phenyl}-6-(4-Methoxyphenyl)-1,3,5-triazin (INCI :Bis-Ethylhexyloxyphenol Methoxyphenyl Triazin) und
b) Propan und/oder Butan,
**dadurch gekennzeichnet, dass** die Zubereitung 2-Ethylhexyl-2-cyano-3,3-diphenyl-acrylat enthält und frei ist von Titandioxid, Zinkoxid und 3-(4-Methylbenzyliden)campher.

2. Kosmetische Emulsion nach Anspruch 1, **dadurch gekennzeichnet, dass** die Zubereitung 4-(tert.-Butyl)-4'-methoxydibenzoylmethan und/oder 2-(4'-Diethylamino-2'-hydoxybenzoyl)-benzoesäurehexylester enthält.

3. Kosmetische Emulsion nach einem der vorhergehenden Ansprüche, **dadurch gekennzeichnet, dass** die Zubereitung eine Mischung aus Propan/Butan enthält.

4. Kosmetische Emulsion nach einem der vorhergehenden Ansprüche, **dadurch gekennzeichnet, dass** die Zubereitung 2,4-Bis-{[4-(2-ethyl-hexyloxy)-2-hydroxyl-phenyl}-6-(4-methoxyphenyl)-1,3,5-triazin in einer Menge von 0,5 bis 5 Gewichts-%, bezogen auf das Gesamtgewicht der Zubereitung, inklusive Propan/Butan, enthält.

5. Kosmetische Emulsion nach einem der vorhergehenden Ansprüche, **dadurch gekennzeichnet, dass** die Zubereitung Propan/Butan in einer Gesamtmenge von 15 bis 45 Gewichts-%, bezogen auf das Gesamtgewicht der Zubereitung, enthält.

6. Kosmetische Emulsion nach einem der vorhergehenden Ansprüche, **dadurch gekennzeichnet, dass** das Gewichtsverhältnis von 2,4-Bis-{[4-(2-ethyl-hexyloxy)-2-hydroxy]-phenyl}-6-(4-methoxyphenyl)-1,3,5-triazin zur Gesamtmenge an Propan/Butan von 0,01 bis 0,2 beträgt.

7. Kosmetische Emulsion nach einem der vorhergehenden Ansprüche, **dadurch gekennzeichnet, dass** die Zubereitung einen oder mehrere weitere UV-Filter enthält, gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenme-thyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzottiazol-2-yl)-4-methyl-6-(2-mothyl-3-[1,3,3,3-tetramethyl-1-[(trimethylsilyl)oxy]disiloxanyl]propyl]-phenol;3-Benzylidencampher; Ethylhexylsalicylat; Terephthalidendicamphersulfonsäure; 4-(Dimethylamino)-benzoesäure(2-ethylhexyl)-ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidetriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-(anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine.

8. Kosmetische Emulsion nach einem der vorhergehenden Ansprüche, **dadurch gekennzeichnet, dass** die Zubereitung in Form einer O/W-Emulsion vorliegt, die einen oder mehrere O/W-Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Ceteareth-20, PEG-40 Stearat, Natriumcetearylsulfat enthält.

9. Kosmetische Emulsion nach einem der vorhergehenden Ansprüche, **dadurch gekennzeichnet, dass** die Zubereitung als weitere Inhaltsstoffe eine oder mehrere Verbindungen gewählt aus der Gruppe der Verbindungen alpha-Liponsäure, Folsäure, Phytoen, D-Biotin, Coenzym Q10, alpha-Glucosylrutin, Carnitin, Carnosin, Polydocanol, natürliche und/oder synthetische Isoflavonoide, Flavonoide, Kreatin, Kreatinin, Taurin, Coffein, Menthol, β-Alanin, Tocopherylacetat, Harnstoff; Hyaluronsäure; Dihydroxyaceton; 8-Hexadecen-1,16-dicarbonsäure, Glycyrrhetinsäure, Glucosylglyceride und/oder Licochalcon A enthält

## Claims

1. Cosmetic emulsion in the form of an aerosol spray, comprising
a) 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine) and
b) propane and/or butane,
**characterized in that** the preparation comprises 2-ethylhexyl 2-cyano-3,3-diphenylacrylate and is free from titanium dioxide, zinc oxide and 3-(4-methylbenzylidene)camphor.

2. Cosmetic emulsion according to Claim 1, **characterized in that** the preparation comprises 4-(tert-butyl)-4'-methoxydibenzoylmethane and/or hexyl 2-(4'-diethylamino-2'-hydroxybenzoyl)benzoate.

3. Cosmetic emulsion according to either of the preceding claims, **characterized in that** the preparation comprises a mixture of propane/butane.

4. Cosmetic emulsion according to any of the preceding claims, **characterized in that** the preparation comprises 2,4-bis{[4-(2-ethylhexyloxy)-2-hydroxy]phenyl}-6-(4-methoxyphenyl)-1,3,5-triazine in an amount from 0.5 to 5% by weight, based on the total weight of the preparation including propane/butane.

5. Cosmetic emulsion according to any of the preceding claims, **characterized in that** the preparation comprises propane/butane in a total amount from 15 to 45% by weight, based on the total weight of the preparation.

6. Cosmetic emulsion according to any of the preceding claims, **characterized in that** the ratio by weight of 2,4-bis{[4-(2-ethylhexyloxy)-2-hydroxy]phenyl}-6-(4-methoxyphenyl)-1,3,5-triazine to the total amount of propane/butane is from 0.01 to 0.2.

7. Cosmetic emulsion according to any of the preceding claims, **characterized in that** the preparation comprises one or more further UV filters, selected from the group of compounds comprising phenylene-1,4-bis(2-benzimidazyl)-3,3',5,5'-tetrasulfonic acid salts; 2-phenylbenzimidazole-5-sulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-benzylidenecamphor; ethylhexyl salicylate; terephthalidenedicamphorsulfonic acid; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone; homomenthyl salicylate; 2-ethylhexyl 2-hydroxybenzoate; dimethicodiethylbenzalmalonate; 3-(4-(2,2-bisethoxycarbonylvinyl)phenoxy)propenyl)methoxysiloxane / dimethylsiloxane copolymer; dioctylbutylamidotriazone (INCI: Diethylhexyl Butamido Triazone); 2,4-bis[5-1(dimethylpropyl)benzoxazol-2-yl-(4-phenyl)imino]-6-(2-ethylhexyl)imino-1,3,5-triazine with CAS No. 288254-16-0; tris(2-ethylhexyl) 4,4',4"-(1,3,5-triazine-2,4,6-triyltriimino)trisbenzoate (also: 2,4,6-tris[anilino(p-carbo-2'-ethyl-1'-hexyloxy)]-1,3,5-triazine (INCI: Ethylhexyl Triazone); 2,4,6-tribiphenyl-4-yl-1,3,5-triazine; merocyanines.

8. Cosmetic emulsion according to any of the preceding claims, **characterized in that** the preparation is in the form of an O/W emulsion which comprises one or more O/W emulsifiers selected from the group of compounds comprising glyceryl stearate citrate, glyceryl stearate (self-emulsifying), stearic acid, stearate salts, polyglyceryl-3 methylglucose distearate, ceteareth-20, PEG-40 stearate and sodium cetearyl sulfate.

9. Cosmetic emulsion according to any of the preceding claims, **characterized in that** the preparation comprises, as further ingredients, one or more compounds selected from the group of compounds comprising alpha-lipoic acid, folic acid, phytoene, D-biotin, coenzyme Q10, alpha-glucosylrutin, carnitine, carnosine, polydocanol, natural and/or synthetic isoflavonoids, flavonoids, creatine, creatinine, taurine, caffeine, menthol, β-alanine, tocopheryl acetate, urea, hyaluronic acid, dihydroxyacetone, 8-hexadecene-1,16-dicarboxylic acid, glycyrrhetinic acid, glucosyl glycerides and/or licochalcone A.

## Revendications

1. Émulsion cosmétique, qui est présente sous la forme d'une pulvérisation en aérosol, contenant :
a) de la 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) et
b) du propane et/ou du butane,
**caractérisée en ce que** la préparation contient de l'acrylate de 2-éthylhexyl-2-cyano-3,3-diphényle et est exempte de dioxyde de titane, d'oxyde de zinc et de 3-(4-méthylbenzylidène)camphre.

2. Émulsion cosmétique selon la revendication 1, **caractérisée en ce que** la préparation contient du 4-(tert.-butyl)-4'-méthoxydibenzoylméthane et/ou de l'ester hexylique de l'acide 2-(4'-diéthylamino-2'-hydroxybenzoyl)-benzoïque.

3. Émulsion cosmétique selon l'une quelconque des revendications précédentes, **caractérisée en ce que** la préparation contient un mélange de propane/butane.

4. Émulsion cosmétique selon l'une quelconque des revendications précédentes, **caractérisée en ce que** la préparation contient de la 2,4-bis-{[4-(2-éthyl-hexyloxy)-2-hydroxy]-phényl}-6-(4-méthoxyphényl)-1,3,5-triazine en une quantité de 0,5 à 5 % en poids, par rapport au poids total de la préparation, y compris le propane/butane.

5. Émulsion cosmétique selon l'une quelconque des revendications précédentes, **caractérisée en ce que** la préparation contient du propane/butane en une quantité totale de 15 à 45 % en poids, par rapport au poids total de la préparation.

6. Émulsion cosmétique selon l'une quelconque des revendications précédentes, **caractérisée en ce que** le rapport en poids entre la 2,4-bis-{[4-(2-éthyl-hexyloxy)-2-hydroxy]-phényl}-6-(4-méthoxyphényl)-1,3,5-triazine et la quantité totale de propane/butane est de 0,01 à 0,2.

7. Émulsion cosmétique selon l'une quelconque des revendications précédentes, **caractérisée en ce que** la préparation contient un ou plusieurs filtres UV supplémentaires, choisis dans le groupe constitué par les composés sels de l'acide phénylène-1,4-bis-(2-benzimidazyl)-3,3'-5,5'-tétrasulfonique ; sels de l'acide 2-phénylbenzimidazole-5-sulfonique ; 1,4-di(2-oxo-10-sulfo-3-bornylidéne-méthyl)-benzène et ses sels ; sels de l'acide 4-(2-oxo-3-bornylidène-méthyl)benzène-sulfonique ; sels de l'acide 2-méthyl-5-(2-oxo-3-bornylidène-méthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-benzylidène-camphre ; salicylate d'éthylhexyle ; acide téréphtalidène-dicamphre-sulfonique ; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; salicylate d'homomenthyle ; 2-hydroxybenzoate de 2-éthyl-hexyle ; benzalmalonate de diméthicodiéthyle ; copolymère de 3-(4-(2,2-bis-éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine de n° CAS 288254-16-0) ; ester tris(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; 2,4,6-tribiphényl-4-yl-1,3,5-triazine, mérocyanine.

8. Émulsion cosmétique selon l'une quelconque des revendications précédentes, **caractérisée en ce que** la préparation se présente sous la forme d'une émulsion H/E, qui contient un ou plusieurs émulsifiants H/E choisis dans le groupe constitué par les composés stéarate-citrate de glycéryle, stéarate de glycéryle (auto-émulsifiant), acide stéarique, sels de stéarate, distéarate de polyglycéryl-3-méthylglycose, cétéareth-20, stéarate de PEG-40, sulfate de cétéaryle sodique.

9. Émulsion cosmétique selon l'une quelconque des revendications précédentes, **caractérisée en ce que** la préparation contient en tant que composants supplémentaires un ou plusieurs composés choisis dans le groupe constitué par les composés acide alpha-liponique, acide folique, phytoène, D-biotine, coenzyme Q10, alpha-glucosylrutine, carnitine, carnosine, polydocanol, isoflavonoïdes naturels et/ou synthétiques, flavonoïdes, créatine, créatinine, taurine, caféine, menthol, β-alanine, acétate de tocophéryle, urée, acide hyaluronique, dihydroxyacétone, acide 8-hexadécène-1,16-dicarboxylique, acide glycyrrhétinique, glucosylglycéride et/ou licochalcone A.
